(19) **Europäisches Patentamt**

**European Patent Office**

**Office européen des brevets**

(11) **EP 0 706 346 B1**

(12) **EUROPEAN PATENT SPECIFICATION**

(45) Date of publication and mention
of the grant of the patent:
**31.05.2000 Bulletin 2000/22**

(51) Int. Cl.[7]: **A61B 10/00**
// G01N33/76, G01N33/74

(21) Application number: **94921625.3**

(22) Date of filing: **24.06.1994**

(86) International application number:
**PCT/EP94/02068**

(87) International publication number:
**WO 95/01128 (12.01.1995 Gazette 1995/03)**

(54) **METHODS FOR MONITORING FERTILITY STATUS**

TESTVERFAHREN ZUR ÜBERWACHUNG DER FRUCHTBARKEITZUSTANDES

PROCEDES D'ESSAIS DESTINES A DETERMINER L'ETAT DE FERTILITE

(84) Designated Contracting States:
**AT BE CH DE DK ES FR GB GR IE IT LI NL PT SE**

(30) Priority: **02.07.1993 EP 93305220**

(43) Date of publication of application:
**17.04.1996 Bulletin 1996/16**

(60) Divisional application:
**99121753.0 / 0 983 748**

(73) Proprietor: **UNIPATH LIMITED**
**Basingstoke, Hampshire RG24 OPW (GB)**

(72) Inventors:
- CATT, Michael
  **Northampton NN8 5XG (GB)**
- MUNDILL, Paul, Henry, Charles
  **Northampton NN10 9HH (GB)**
- ZHANG, Zhi, Gang
  **Bedford MK41 9AL (GB)**

(74) Representative:
**Butler, David John**
**Unilever PLC**
**Patent Division**
**Colworth House**
**Sharnbrook**
**Bedford MK44 1LQ (GB)**

(56) References cited:
| | |
|---|---|
| **EP-A- 0 367 615** | **EP-A- 0 385 621** |
| **WO-A-94/04926** | **DE-U- 8 805 565** |
| **US-A- 4 151 833** | **US-A- 5 043 888** |

- **GYNECOLOGIC AND OBSTETRIC INVESTIGATION, vol.29, 1990, BASEL pages 207 - 210 PAZ ET AL. 'Determination of urinary luteinizing hormone for prediction of ovulation'**
- **'Proc Xth Int'l Congress on Fertility and Sterility' 1981 , MTB LTD cited in the application Collins et al. : see page 19 - page 33**

**EP 0 706 346 B1**

## Description

This invention relates to methods, devices and test kits for use in monitoring fertility status in female mammals especially humans.

The invention is particularly, although not solely, concerned with simple practical procedures that can readily be applied by unskilled persons, e.g. in the home, to provide reliable information concerning fertility status as an aid to contraception. An important objective of the invention is to provide such information while avoiding the necessity for tests to be conducted on a frequent (eg. daily) basis throughout every ovulation cycle. The necessity for regular, e.g. daily, testing throughout the cycle has characterised many ovulation cycle monitoring systems previously proposed.

The invention may also be used by persons wishing to enhance the likelihood of conception, by providing an indication of the time during the ovulation cycle when fertilization is most likely to occur.

To provide reliable information concerning fertility status, the user must be given adequate warning of the onset of the fertile phase in the cycle. A wide variety of techniques have proposed in the art, some relying on the monitoring of one or more parameters which alter as the event of ovulation approaches. Typical parameters which have been invoked are the concentration of a body fluid analyte, such as estradiol and metabolites thereof, for example estrone-3-glucuronide (E3G). Other parameters that have been used are basal body temperature (which can only provide predictive information of use in subsequent cycles) and various physiological changes such as the characteristics of vaginal mucous.

Many excellent academic studies have been carried out using such parameters. Such studies have established how these parameters can be correlated with the fertility status of an average member of a large population sample. An example is Collins et al (1981), Proc. Xth International COngress on Fertility and Sterility, Publ MTP Ltd, p 19-33. An underlying objective in many such studies is to promote conception in individuals previously regarded as being infertile.

However, when attempting to develop a practical monitoring system suitable for use by individuals, it is found that many individual subjects do not conform to the average in terms of cycle length and/or the duration and timing of the fertile phase. The extent of variation from one individual to another, and indeed, from one cycle to another in the same individual, renders average population data too unreliable for consistent practical use.

Understandably, because the severe consequence of imperfect advice concerning fertility status may be an unwanted pregnancy, the tendency has been to exercise extreme caution and to require testing of the relevant parameter or parameters throughout the cycle, and particularly right from the onset of the cycle (onset of menses). From the individual user's point of view, it would be advantageous if the necessity for such constant testing could be avoided and, instead, for the testing to be performed over a comparatively brief portion of each cycle. Not merely may this benefit the user in terms of convenience, but the cost of the method may also be reduced if the method utilises disposable testing devices and only a few such disposable testing devices are required each month.

An example of a system for detecting the onset of ovulation, using water-swellable polymer pellets to "measure" the water content of vaginal mucus, whic, apparently increases at the time of ovulation, is described in US 4151833 (Polishuk). It is stated that the peak variation in the size of the pellets, as a result of the absorption of water from cervical mucus, is closely related to the LH surge and the variation in basal body temperature. From the experimental data provided in US 4151833 (Figure 8), it appears that the pellet diameter is indeed very closely related to the timing of the LH surge, and in consequence the system proposed cannot in practice provide a reliable warning of the onset of ovulation earlier than that obtainable from a knowledge of the LH concentration.

In EP-A-385621 (Coley et al/Unilever) the defects of ovulation cycle monitoring systems which rely primarily on the change in BBT to estimate the time of ovulation are described, and we propose therein a system which uses regular BBT measurement in combination with a knowledge of other parameters, particularly the measurement of certain urinary hormone levels. A particular proposal is that BBT is measured daily throughout each cycle and is used to estimate the timing of fertility status changes in a forthcoming cycle. During the course of this forthcoming (predicted) cycle, urinary hormone levels are checked at certain times to confirm that the progress of the cycle, as predicted from the previous BBT knowledge, is consistent. Particular hormones selected are E3G, P3G and LH. It is suggested that the level of urinary E3G is measured on at least one day during the interval from day 5 to 7 of the predicted cycle, and again on at least one day during the interval from day 10 to day 15 of the predicted cycle. According to the example in EP 385621, it is sufficient for the hormone level to be either "high" or "low" relative to a threshold value. The emphasis throughout EP 385621 is that occasional hormone level measurements are used to supplement a monitoring system which relies on BBT measurement. There is no suggestion that hormone measurements alone could provide the basis for a reliable fertility monitoring system personalised for an individual subject.

WO-A-9404926 (Catt et al/Unilever) citable under Article 54(3) EPC discloses a method of monitoring the status of a current ovulation cycle of an individual human female subject, involving testing of the body fluid concentration of an analyte of significance in relation to the status of the ovulation cycle, such as urinary E3G, during at least part of the pre-ovulation phase of the current ovulation cycle of the individual subject, and identification from the results of such testing of an analyte concentration charge indicative of imminent ovulation, relative to an analyte concentration reference value that has been adapted to the individual human subject on the basis of analyte concentration test data obtained from the individual human subject during one or more previous ovulation cycles.

An objective of the present invention is to provide a system for monitoring the fertility status of an individual subject, which provides sufficient warning of the onset of the fertile phase to enable contraceptive advice to be given and which can be personalised to the individual subject, while being based solely on body fluid analyte measurements. The inherent unreliability, or limited usefulness, of other measuring systems (such as BBT) can thereby be avoided. A further objective is to avoid the use of average data obtained from population studies, with its inherent risk that in an individual subject, the parameter under test can fluctuate considerably from the population norm.

A further objective is to provide the option of basing an effective monitoring system solely on the measurement of a single body fluid analyte, such as estradiol or a metabolite thereof. Other advantages of the invention will be apparent from the following description.

Another objective of the invention is to provide a testing regime which is a good balance between the desire to minimise the testing burden on the user and the need to give the user worthwhile advice about the fertility status.

The invention provides a method of monitoring the fertility status of an individual female mammalian subject, wherein the concentration of an analyte in body fluid obtainable from the subject is tested at least once during the interval spanning days 1 to 7 inclusive of the current cycle and also later in the current cycle to determine whether a concentration change indicative of imminent ovulation is occurring or has occurred, characterised in that:

a) the mean numerical day on which actual ovulation has occurred during one or more previous ovulation cycles in the subject is determined;

b) the at least one day test conducted during the interval spanning days 1 to 7 is used to establish a reference concentration value or test signal for said analyte for the current cycle; and

c) the later testing in the current cycle comprises a series of tests for said analyte conducted during a period of days commencing at least 5 numerical days in advance of said mean numerical ovulation day, and the concentration value or test signal from each of said later tests is compared to the reference concentration value or test signal.

For the purposes of illustration only, the invention will be described in relation to the measurement of urinary analytes, and especially "E3G" (estrone-3-glucuronide) and "LH" (luteinizing hormone).

In addition to estrone-3-glucuronide already mentioned, estradiol metabolites that can also be assayed for the purposes of the invention include estradiol-3-glucuronide, estradiol-17-glucuronide, estriol-3-glucuronide, estriol-16-glucuronide and (principally for non-human subjects) estrone-3-sulphate. As will be appreciated from the following description, the invention can readily be applied to data derived from the measurement of body fluid concentrations of other analytes of significance in relation to the status of the ovulation cycle. Generally, the most suitable analytes are hormones and their metabolites. Follicle stimulating hormone (FSH) is an example. Examples of alternative body fluids, which are relatively accessible, are saliva, crevicular fluid, sweat, sebum, tears and vaginal fluid. In principle internal fluids, such as blood, can be used but are generally not preferred because they can only be accessed by invasive techniques.

The skilled reader will also appreciate that the body fluid "concentration" of the chosen analyte or analytes need not be measured in absolute terms, although this can of course be done if desired. Generally, it will be sufficient to assay an analyte in a manner which yields a signal, convertible to numerical data, related to the actual concentration, so that such data can be compared with similar data obtained at a different stage in the cycle to determine whether or not a significant change in actual concentration has occurred. Accordingly, where the specification and claims below refer to the "concentration" of an analyte, this expression should be interpreted broadly.

An important aspect of the invention is a method of monitoring the current fertility status of an individual human female, involving testing of the body fluid concentration of estradiol or a metabolite thereof and comparing the test result with a reference value or signal to ascertain whether an elevated concentration indicative of imminent ovulation is present, wherein the reference value or signal for the current ovulation cycle is established by testing the body fluid concentration in the same individual at least once during the interval spanning days 1 to 7 inclusive of the current cycle.

The invention therefore concerns a method of monitoring the fertility status of an individual female mammalian subject, involving testing of the body fluid concentration of at least one analyte of significance in relation to the status of the ovulation cycle during the pre-ovulation phase, wherein testing for said analyte is conducted at least once during the interval spanning days 1 to 7 inclusive of the current cycle calculated from the onset of menses (day 1 being the day on which menstruation is first observed), to establish a reference concentration value or signal for said analyte in the current cycle, and thereafter testing is conducted at least once (generally repeatedly, e.g. daily) prior to a day on which ovulation is likely to occur during the cycle, analyte concentration values or signals obtained during said later or repeated testing being compared with the reference concentration value or signal to determine whether a concentration change indicative of imminent ovulation is occurring or has occurred since the previous test.

In a preferred embodiment, the invention provides a method of monitoring the fertility status of an individual female subject, involving testing of the body fluid concentration of at least one analyte of significance in relation to the status of the ovulation cycle during the pre-ovulation phase, wherein testing for said analyte is conducted at least once during the interval spanning days 1 to 7 inclusive calculated from the onset of menses (day 1 being the day on which menstruation is first observed), to establish a reference concentration value or signal for said analyte in the current cycle, and then testing is conducted at least once (generally repeatedly, e.g. daily) during a period of days commencing at least 5, and more preferably at least 6, numerical days in advance of the mean numerical day on which actual ovulation has occurred over one or more previous ovulation cycles in the same individual subject, analyte concentration values or signals obtained during said period of days being compared with the reference concentration value or signal to determine whether a concentration change indicative of imminent ovulation is occurring or has occurred since the previous test. Generally, the repeated testing need not be commenced earlier than about 9 days in advance of the mean ovulation day.

Preferably, the concentration reference value is established from test(s) conducted during the interval spanning days 4 to 7 inclusive, more preferably from test(s) conducted on day 5 and/or day 6, and most preferably from a single test conducted on day 6.

A significant change in analyte concentration indicative of imminent ovulation, particularly appropriate when the analyte is estradiol or a metabolite thereof, will generally be noted when the ratio of the reference concentration [r] to the test concentration [i] meets the following criteria:

$$1.5 \le \frac{[i]}{[r]} \le 2.5$$

In particular, especially when the analyte is E3G and the reference value is established on day 6:

$$\frac{[i]}{[r]} \ge 2$$

If the chosen assay format by means of which concentration data is obtained yields a signal which is inversely proportional to actual concentration, as may be the case in a competition assay, it will be appreciated by the skilled reader that the relationship between [i] and [r] signals will be the inverse of those given above.

It is generally envisaged that there will be a gap of at least one day, and more usually several days, between establishment of the concentration reference value and the commencement of repeated testing, during which gap no testing need be conducted. Thus, in the ideal situation, the user performs a single test at an early stage of the cycle, eg on day 6, and several days later commences a relatively brief schedule of repeated, eg daily testing, which is terminated after sufficient information has been derived to identify the fertile phase, preferably including an indication of the end of the fertile phase in that cycle. Typically this termination of testing will be on the day of LH surge, or within a few days thereafter, so that the remainder of the cycle is test-free.

Conveniently, the body fluid can be urine. A very suitable analyte is therefore estradiol or a metabolite thereof, such as estrone-3-glucuronide.

Preferably, in one embodiment of the invention, the mean ovulation day is derived from data collected during at least 3, and more preferably at least 5, consecutive previous cycles.

Ideally, the mean ovulation day used to calculate the time interval for the purposes of the current cycle is derived from data obtained during at least the immediately preceding cycle.

A particularly convenient method involves the determination of the mean ovulation day from data obtained from a "rolling" reference base consisting of a fixed number of consecutive cycles immediately preceding the current cycle. Preferably this rolling reference base consists of the immediately preceding 3 to 12 cycles, more preferably the immediately preceding 5 or 6 cycles. By having such a rolling reference base, any progressive "drift" in the occurrence of ovulation in the individual concerned can be picked up and accounted for in the allocation of the next repeated testing commencement day.

The invention can be performed using a test kit comprising one or more testing devices for determining the concentration (in relative or absolute terms) of said at least one analyte in said body fluid, together with instructions advising the user to commence said testing during said time interval, and means enabling a user to derive said time interval and/or a precise testing commencement day from knowledge of the numerical day on which actual ovulation occurred during at least one previous ovulation cycle of the user.

Another aspect of the invention, which may be combined to advantage with any method as set forth above, involves:

a) providing the user with a plurality of disposable body fluid testing devices, said plurality preferably being at least 7, but preferably not greater than 12; and

b) directing the user to use all of said provided testing devices during a single ovulation cycle, in accordance with a predetermined testing schedule, irrespective of whether an indication of imminent ovulation has been obtained before all of said provided testing devices have been used.

Preferably, the user is directed to perform one test on day 6, and to use all of the remaining testing devices on a daily basis during the repeated testing period.

The invention can be performed using a kit comprising a plurality of disposable body fluid testing devices, together with means for reading and interpreting the results of tests performed using said testing devices.

Optionally a replenishment pack of disposable body fluid testing devices for use in any of the methods as set forth above is provided, with directions to the user to use all of said contained disposable testing devices during the course of a single ovulation cycle. Preferably the pack contains not more than 12 testing devices, more preferably at least 7 but not more than 10 devices.

By requiring the user to use all of a numerically-small single batch or set of disposable testing devices per cycle, there are advantages both for the user and for the manufacturer of the devices. The user benefits because the "monthly" testing schedule is simplified - there is no need for a decision to be taken on when to stop the repeated testing, or about using up during subsequent cycles testing devices left over from earlier cycles. For the manufacturer, there is assurance that data for each cycle is derived from a single batch of testing devices thus eliminating problems of standardisation that might otherwise arise, and reducing the complexity of any monitor required to interpret the test data. No activity by the user is required to ensure calibration of the assays. The disposable testing devices can be supplied in standard "monthly" replenishment packs, streamlining the packaging operation. Because the problem of "leftover" testing devices is eliminated, one possible cause for customer enquiries is also avoided.

An advantage of the methods of the present invention is that effective monitoring of the ovulation cycle can be achieved using data derived solely from the measurement of body fluid analyte concentration(s). It is unnecessary to combine this data with other parameters. In particular, there is no need to supplement this data with routine measurement of basal body temperature.

By adopting a concentration reference value from data in the early part of the current cycle, the methods of the invention avoid the need for calibration and ensure that the base-line reference is personal to the subject under test. This leads to a clearer indication of the significant pre-ovulation concentration change, compared to previously proposed methods based on day-to-day measurements.

The analyte chosen for providing the warning of imminent ovulation is not critical to the invention, provided that the analyte exhibits a detectable concentration change within the time interval between the commencement of testing (as determined herein) and a safe time in advance of actual ovulation in the current cycle.

The invention can be applied in any method of monitoring the status of a current ovulation cycle of an individual human female subject involving the measurement of a body fluid analyte of significance in relation to the status of ovulation cycle and which exhibits a detectable change during the pre-ovulation phase of the cycle occurring at least 2 and more preferably at least 3 days in advance of the day of actual ovulation.

The following description is provided, by way of example only, in relation to the urinary hormones E3G, luteinizing hormone (LH), and pregnanediol-3-glucuronide (P3G), although it will be readily appreciated that the principles of the method can be used in relation to other biochemical markers, for example the hormones estradiol and progesterone, found for example in the blood or in saliva. The method of the invention may be used in combination with observations of other physiological signs of the level of fertility in a female, of which she is aware, or can readily be made aware of, e.g. markers in other body fluids.

Ovulation day can be determined by any of the known chemical or physiological parameters, although a preferred method is by measuring the level of LH. Once the LH surge has been detected, it can be said that ovulation is imminent. Also, the day of the cycle on which ovulation has occurred can be noted for future reference. If the LH surge is detected, and hence the day of ovulation accurately pinpointed, it can be indicated to the user with a very high degree of certainty that the subject will no longer be fertile four days hence (3 days after ovulation). For practical purposes, a urinary LH concentration of 20 mIU/ml can be regarded as a universal threshold indicative of the LH surge under virtually all circumstances.

The expression "LH surge" is used herein to mean the dramatic rise in LH concentration that precedes the event of ovulation. In the art, reference is made also to "LH max", i.e. the peak concentration of LH. In the majority of individuals, these are for all practical purposes simultaneous, when the cycle is monitored on a day-by-day basis. However, in a few individuals, perhaps 20% of the population, the actual peak concentration of LH is not observed until the day following the main concentration rise. For the purposes of the invention, we prefer to use the observable rise as the critical parameter.

Alternatively, or in addition, the end of the fertile phase can be declared on the basis of knowledge of the estradiol (or metabolite thereof) concentration, in the current cycle. Conveniently, this may be declared on a set day following a peak concentration value. Because the peak concentration of urinary E3G, for example, appears to be a less readily detectable event than the LH surge, the E3G "peak" may be defined by reference to a threshold value, determined for example by the relationship:

$$\frac{[i]}{[r]} > 2.5, \text{ preferably} \geq 3$$

the "peak" being taken to occur on the day when this relationship is first satisfied during the testing regime adopted in the current cycle. The inverse relationship will apply if the E3G signal in inversely proportional to actual concentration. In some instances this may be the same day as the significant E3G rise indicative of imminent ovulation is detected. When the E3G "peak" has been detected, the fertile phase can be assumed to end on the sixth, or more safely the seventh or eighth, day later. In this embodiment, the invention provides the option of a method of monitoring fertility in the current cycle based solely on data derived from estradiol/metabolite assays.

Another method for predicting the end of the fertile period (though not so accurately the day of ovulation) is to measure the levels of the urinary hormone P3G. P3G has a relatively low level in urine until the start of the luteal phase, at which point its level rises fairly sharply. Therefore, once an elevated level of P3G is detected, it can be indicated to the user that the luteal phase of the cycle - ie. the terminal infertile period - has commenced. An elevated level of urinary P3G can be based on data taken during the current and/or one or more preceding cycles. An "elevated" P3G level can be recorded, for example, when either the level of P3G detected is greater than the sum of the four previous recorded levels of P3G in the same menstrual cycle, or greater than 3500 ng/ml, whichever of these two thresholds is lower and is first achieved. Once an "elevated" P3G level is recorded, the subject can be advised that she is infertile for the remainder of that cycle.

If desired, the detection of either LH or P3G can be used as a trigger to indicate that the subject is no longer fertile until the end of the cycle, with one hormone acting as a "back up" to the other. However, it is preferred that the detection of LH be used as a primary indicator of whether ovulation has or is about to occur, since the detection of LH lends itself to more accurate determination of the exact ovulation day than the use of P3G. Methods of detecting body fluid analytes, such as urinary hormone metabolites, suitable for the purposes of this method, are well known to those skilled in the art. In a preferred embodiment, the analyte is detected by assay methods and devices as described in our UK patent GB 2204398 and our European patent application EP-A-383619, the contents of which are incorporated herein by reference.

Where the method of the invention relies on measurement of a urine component, this must be done on a urine sample. A variety of immunoassay techniques are available which enable urine components to be measured. A wide variety of solid phase testing devices such as dipsticks and chromatographic strips have been described in the literature, and can readily be adapted for use in determining urinary analytes. The device should at least be capable of indicating relative levels of analyte, eg. E3G, in threshold bands. Examples of simple assay technology that can readily be adapted for use in the home is described, for example, in EP 0225054, EP 0183442, EP 0186799 and GB 2204398, the disclosures of these specifications being incorporated herein by reference. Disposable assay strips such as those described in GB 2204398 which simply require to be contacted with urine and which provide an assay result in semi-qualitative form, eg. by means of a series of test zones on the strip which are progressively positive at higher urinary analyte levels, can be used. Multiple strips that respond at different analyte thresholds can be used, rather than a single strip. Alternatively, a visually readable quantitative assay can be based on progression of a visible, eg. coloured, region or "front" over a surface (eg. radial diffusion), using for example an enzyme-labelled assay.

In a more sophisticated embodiment of the invention, a recording device is provided which incorporates means for reading the result of the urine assay, eg. by measuring the absorbance by or fluorescence from an assay strip. This may enable a more precise numerical indication to be given of the analyte level, and further enhance the accuracy of the method.

In an embodiment of the invention in which two or more analytes are measured simultaneously, such measurement can if desired be performed using a single body fluid testing device, eg. a device incorporating multiple assay strips, or a single strip capable of independently detecting the level of the different analytes.

The detailed electronics of a recording device capable of assimilating, remembering and handling analyte concentration data, as well as providing the preferred electronic features of the device discussed herein, and predicting future cycles on the basis of such data, can readily be provided by those skilled in the electronics art once they have been advised of the factors that such a device must take into consideration, and the information that the device must provide for the user. Such detailed electronics do not form part of the invention. However, by way of example only, the basic functions that may be required in such a device are outlined in Figure 3 of the accompanying drawings and described briefly below.

By way of example only, practical aspects of the invention are described below with reference to the accompanying drawings, of which:

Figure 1 of the accompanying drawings illustrates an ovulation cycle monitoring device for use in accordance with the invention, together with an associated urine sample testing device.

Figure 2 shows the urine testing device in greater detail.

Figure 3 shows, in schematic form, the basic functions that may be required in an electronic monitor for use in accordance with the invention.

Referring to Figure 1, the urine sample testing device comprises a flat elongate casing 100 having a locating means, represented by ridge 101 on its lower surface 102. Projecting from one end of the casing is a bibulous sample receiving member 103.

The monitor comprises a casing 110 having a recess 111 in its upper surface 112 to accommodate the casing 100 of the testing device. Recess 111 incorporates a locating slot 113 into which the locating ridge 101 on the casing of the testing device can be inserted to positively locate the testing device in relation to a reading window 114 in the recess. Casing 110 contains means (not shown) such as a fluorescence reader or optical density reader to measure the result of a urinary analyte concentration assay performed using the testing device, according to the labelling system chosen to reveal the assay result.

The sloping front face 115 of the monitor casing incorporates a large window 116 through which information can be conveyed to the user eg. by means of an LED display or other visual output. This information can be provided in a variety of forms, such as an indication of a calender and the need to perform urine tests, and an indication of the current status of the ovulation cycle. The sloping face 115 of the casing also incorporates a button 117 which the user can press to indicate the commencement of an ovulation cycle and to start the monitor processing information relative to that cycle.

Information can be conveyed to the user by means of a liquid crystal or LED display, for example. If desired, information on the state of fertility can be conveyed by a simple visual indication, eg a combination of colours showing, for example, green for infertile and red for fertile. Optionally, another signal, e.g. yellow, for any intermediate stage when conception is less likely but still possible, may be shown. Especially if the device is intended primarily as an aid to contraception, it should "fail safe" by showing a "fertile" signal.

The invention can be performed using a kit for monitoring the ovulation cycle of a female mammal, comprising a monitoring device as set forth above, together with at least one tasting device capable of being used to measure the level of one or more urine components. It is envisaged that the monitoring device will generally be of a relatively durable nature and capable of being used over a considerable number of cycles. The testing devices for measuring the urine components are preferably disposable after individual use, and it is therefore envisaged that the user of the monitoring device will need to replenish the testing devices.

In general the monitor will be battery-powered, and incorporates in side 118 of the casing an access point such as a removable cover 119 to permit batteries to be inserted and changed.

Referring to Figure 2, the testing device is shown inverted relative to the aspect seen in Figure 1. The locating ridge 101 is now on the upper surface 200. Also in the surface 200 now uppermost is a result window 201. The body of the testing device can incorporate an immunochromatograhic strip (not shown) incorporating all necessary reagents to enable an immunoassay to be formed which detects the presence and concentration of analyte in a urine sample applied to the sample collecting member 103. The result of the assay can be effected by the immobilization of a labelled component, via a sandwich or competition reaction in the presence of analyte in an applied urine sample, the labelled reagent becoming concentrated in a zone revealed through the result window. When the testing device is inverted and located in the recess 111 in the casing of the monitor, the result window is immediately adjacent to the reading window 114 in the monitor and the assay result can be determined. For example, if the label is a fluorescent reagent, the reading means within the monitor can detect and measure fluorescent light output from the accumulated label in the detection zone on the strip to provide a numerically accurate concentration value for the analyte in the urine sample. This information can be processed by the monitor together with calender information resulting from the initiation of the cycle process by the user and historical data which the monitor can retain from previous cycles.

Referring to Figure 3, some of the basic elements which may be required in an electronic monitoring device are seen. The individual features can be entirely conventional, and those familiar with the art of electronics will appreciate that other combinations and arrangements of such features can be employed to achieve the objectives of the invention. For example, so-called "hard-wired" systems, and "neural networks", can be used in place of conventional microprocessors based on "chip" technology. As depicted in Figure 3, the combination essentially comprises:

A reading unit 300 to derive information from a test device, such as a test stick, the reading unit comprising an illuminator 301 and a reader 302 (represented here as a photo diode). The reading unit feeds into a conversion unit 303 to convert the optical signal into a form usable by a microprocessor 304. As an optional feature, a calibration system 305 is provided to convert the signal derived from the reading unit into data corresponding, for example, to an absolute concentration value. A timer, such as a clock 306 is required to regulate measurements within a cycle. The microprocessor 304 processes, memorizes and interprets results in the light of previous events, particularly recorded from previous cycles. The user interface 307 will generally comprise at least means, such as a push button, which the user can operate at the commencement of a cycle to initiate the operation of the device as a whole. The power supply 308 should include means, such as a memory back up capacitor 309, to prevent loss of historical data if it becomes necessary to replace batteries.

Aspects of the invention are illustrated in the following Examples. These relate to the monitoring of the human ovulation cycle.

## EXAMPLE 1

This example sets out a convenient algorithm on which a monitoring method in accordance with the invention can be based. The kit provided to the user comprises a plurality of dual-analyte disposable urine testing devices, capable of assaying urinary E3G and urinary LH in a form readable by a monitor also provided. The monitor can receive each used testing device and determine the urinary concentration of each analyte. This information is stored in the monitor and compared with similar data obtained on subsequent days in the same cycle. The monitor has a menstruation button that the user must press at the start of the cycle, and a display panel or the like to convey information about cycle status, and to indicate to the user when testing should be performed.

a) Algorithm rule structure

The objective is to:

i) identify the position of the LH surge in an individual cycle;
ii) identify a significant increase in E3G concentration in an individual cycle with respect to the E3G concentration on day 6 of that cycle.

The number of tests available each routine month is limited to 8, and a test strategy which will maximise the chances of achieving i) and ii) is adopted.

b) Start-up cycles

In order to establish an adequate initial data base, during the first cycle of use the monitor requires sixteen tests. This is to establish baseline data for the individual. The user presses the menstruation button on the monitor on the morning after her menstruation begins. This day is recorded as day 1 by the monitor. Testing commences on day 8 and continues daily until day 23. This testing is targeted to maximise the chance of observing the LH surge.

At the start of cycle 2 and the start of all following cycles, the menstruation button is pressed as above. From cycle 2 onwards, eight tests only are used for each cycle. All eight tests must be from the same batch and all must be completed. In all cycles from cycle 2 onwards, testing commences on day 6. For cycles 2 and 3, tests two to eight are conducted on consecutive days starting on the typical LH surge day minus four days. From cycle 4 onwards, regarded as the first routine cycle, tests two to eight are conducted in sequence from typical LH surge minus five days. The typical LH surge day is defined as the mean day of LH surge for up to the previous six months.

c) Start of the fertile phase

In cycle 1, the monitor declares the woman fertile from day 6 onwards, as no information about cycle characteristics has been collected. In cycles 2 and 3, the monitor can use the typical position of the LH surge from the previous cycle(s) to determine the start of the fertile phase. However, because of the limited amount of cycle data available, the monitor will still declare a woman fertile during cycles 2 and 3 on day 6 or on typical LH surge minus 7 days, whichever is later.

In subsequent routine cycles, the onset of the fertile phase is set by detection of a significant change in the E3G signal with respect to day 6. When the ratio of the current day's signal for E3G ($S_i$) to the day 6 signal ($S_6$) reaches a set threshold as set forth above, the woman is declared fertile.

d) <u>End of the fertile phase</u>

In normal operation, the end of the fertile phase is defined as the fourth morning after the detection of the LH surge. In the absence of a detectable LH surge during the test sequence, the system declares the end of the fertile phase to be six days after the last test. The rationale for this calculation is as follows. The testing regime is designed to cover the typical position of the LH surge plus one day. Published WHO study data showed within-woman variability of LH surge position to be 1.8 days. Adding five days to the declared fertile period after testing has been completed allows two standard deviations confidence that the LH surge will occur within the assigned fertile period.

In non-normal operation (cycle 1), where the monitor has no information about the typical LH surge position, if this parameter is not detected, the end of the fertile phase is declared on cycle day 28.

## **EXAMPLE 2**

This example uses representative E3G profiles from two women - one known to have low levels of urinary E3G and the other known to have relatively high levels. In the first two columns of each table, 30 days of each cycle are set out in terms of their fertility. The first phase is termed infertile and consists of that portion of the follicular phase during which unprotected intercourse would not be expected to result in conception, followed by a transitional phase during which changes occur that lead to a fertile state and during which a positive signal to indicate the onset of the fertile phase is required. The fertile phase is that phase before and after ovulation during which unprotected intercourse is most likely to result in conception. Its duration before ovulation is dictated entirely by the effective lifetime of sperm, and this, in turn is influenced by factors controlled by the female hormones, especially mucus. The post fertile, luteal phase is that time after which the ovum has left the uterus and conception in the current cycle is no longer possible.

E3G values are given in the third column. These were derived by immunoassay on early morning urine samples collected each day. The immunoassay was a conventional enzyme-labelled-antigen competitive assay. The values given are in ng/ml.

Actual ovulation is taken as 24 hours following the LH surge. These LH values were determined by conventional enzyme-labelled sandwich immunoassay on the same samples, but the values are not included in the table as ovulation date is the essential result.

The algorithm of Example 1 has been applied to each cycle, taking the E3G trigger point as:

$$\frac{[i]}{[\text{day 6}]} \geq 2$$

INDIVIDUAL A

| CYCLE A 1: Start-up cycle | | | | | |
|---|---|---|---|---|---|
| Day | Test | Phase | E3G value | "Red" status | Actual ovulation |
| 1 | | infertile | | | |
| 2 | | infertile | | | |
| 3 | | infertile | | | |
| 4 | | infertile | | | |
| 5 | | infertile | | | |
| 6 | | infertile | | *** | |
| 7 | | infertile | | *** | |

(continued)

| CYCLE A 1: Start-up cycle | | | | | |
|---|---|---|---|---|---|
| Day | Test | Phase | E3G value | "Red" status | Actual ovulation |
| 8 | * | infertile | 1.9 | *** | |
| 9 | * | infertile | 3.1 | *** | |
| 10 | * | infertile | 5.4 | *** | |
| 11 | * | infertile | 2.1 | *** | |
| 12 | * | infertile | 5.3 | *** | |
| 13 | * | infertile | 10.5 | *** | |
| 14 | * | infertile | 7.7 | *** | |
| 15 | * | fertile | 5.2 | *** | |
| 16 | * | fertile | 8.3 | *** | |
| 17 | * | fertile | 6.8 | *** | |
| 18 | * | fertile | 4.3 | *** | LHS + 1 |
| 19 | * | fertile | 4.9 | *** | |
| 20 | * | fertile | 5.3 | *** | |
| 21 | * | postfertile | 3.3 | | |
| 22 | * | postfertile | 4.9 | | |
| 23 | * | postfertile | 6.2 | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |

LH surge was on day 17, therefore repeated testing to commence on day 13 in next cycle.

| CYCLE A 2 | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | * | infertile | 3.5 | | |
| 7 | | infertile | | | |
| 8 | | infertile | | | |

(continued)

| CYCLE A 2 | | | | | |
|---|---|---|---|---|---|
| Day | Test | Phase | E3G value | "Red" Status | Actual Ovulation |
| 9 | | infertile | | *** | |
| 10 | | infertile | | *** | |
| 11 | | infertile | | *** | |
| 12 | | infertile | | *** | |
| 13 | * | infertile | 8.9 | *** | |
| 14 | * | fertile | 14.6 | *** | |
| 15 | * | fertile | 12.6 | *** | |
| 16 | * | fertile | 8.8 | *** | |
| 17 | * | fertile | 15.8 | *** | LHS + 1 |
| 18 | * | fertile | 6.9 | *** | |
| 19 | * | fertile | 6.5 | *** | |
| 20 | | postfertile | | | |
| 21 | | postfertile | | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Mean LHS of cycles A1 and A2 is day "16.5", therefore repeated testing to commence on day 12 in next cycle. | | | | | |

| CYCLE A 3 | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | * | infertile | 1.6 | | |
| 7 | | infertile | | | |

(continued)

| CYCLE A 3 | | | | | |
|---|---|---|---|---|---|
| Day | Test | Phase | E3G value | "Red" Status | Actual Ovulation |
| 8 | | infertile | | | |
| 9 | | infertile | | *** | |
| 10 | | infertile | | *** | |
| 11 | | infertile | | *** | |
| 12 | * | fertile | 6.2 | *** | |
| 13 | * | fertile | 23.6 | *** | |
| 14 | * | fertile | 21.3 | *** | |
| 15 | * | fertile | 8.3 | *** | LHS + 1 |
| 16 | * | fertile | 4.5 | *** | |
| 17 | * | fertile | 3.7 | *** | |
| 18 | * | postfertile | 3.4 | | |
| 19 | | postfertile | | | |
| 20 | | postfertile | | | |
| 21 | | postfertile | | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Mean LHS from cycles A1 to A3: day "15.7". Repeated testing commencement day for first routine cycle: day 10. | | | | | |

| CYCLE A 4<br>First routine cycle | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | * | infertile | 3.1 | | |

(continued)

| CYCLE A 4<br>First routine cycle | | | | | |
|---|---|---|---|---|---|
| Day | Test | Phase | E3G value | "Red" Status | Actual Ovulation |
| 7 | | infertile | | | |
| 8 | | infertile | | | |
| 9 | | infertile | | | |
| 10 | * | infertile | 6.1 | | |
| 11 | * | fertile | 16.7 | *** | |
| 12 | * | fertile | 10.8 | *** | |
| 13 | * | fertile | 22.8 | *** | |
| 14 | * | fertile | 21.3 | *** | LHS + 1 |
| 15 | * | fertile | 9.4 | *** | |
| 16 | * | fertile | 12.2 | *** | |
| 17 | | postfertile | | | |
| 18 | | postfertile | | | |
| 19 | | postfertile | | | |
| 20 | | postfertile | | | |
| 21 | | postfertile | | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Days warning of actual ovulation: 3<br>Mean LHS from cycles A1 to A4: day "15.3".<br>Repeated testing commencement day for next cycle: day 10. | | | | | |

| CYCLE A 5<br>Second routine cycle | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |

(continued)

| CYCLE A 5 Second routine cycle | | | | | |
|---|---|---|---|---|---|
| Day | Test | Phase | E3G value | "Red" Status | Actual Ovulation |
| 4 | | infertile | | | |
| 5 | | infertile | | | |
| 6 | * | infertile | 4.8 | | |
| 7 | | infertile | | | |
| 8 | | infertile | | | |
| 9 | | infertile | | | |
| 10 | * | infertile | 8.5 | | |
| 11 | * | infertile | 7.3 | | |
| 12 | * | infertile | 6.3 | | |
| 13 | * | infertile | 7.0 | | |
| 14 | * | fertile | 11.8 | *** | |
| 15 | * | fertile | 19.3 | *** | |
| 16 | * | fertile | 18.5 | *** | |
| 17 | | fertile | | *** | LHS + 1 |
| 18 | | fertile | | *** | |
| 19 | | fertile | | *** | |
| 20 | | postfertile | | | |
| 21 | | postfertile | | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Days warning of actual ovulation: 3 Mean LHS from cycles A1 to A5: day "15.4". Repeated testing commencement day for next cycle: day 10. | | | | | |

INDIVIDUAL B

| CYCLE B1: Start-up cycle | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | | infertile | | *** | |
| 7 | | infertile | | *** | |
| 8 | * | infertile | 25.1 | *** | |
| 9 | * | infertile | 10.1 | *** | |
| 10 | * | infertile | 16.8 | *** | |
| 11 | * | infertile | 28.2 | *** | |
| 12 | * | infertile | 24.6 | *** | |
| 13 | * | infertile | 28.7 | *** | |
| 14 | * | infertile | 27.7 | *** | |
| 15 | * | infertile | 62.6 | *** | |
| 16 | * | infertile | 68.5 | *** | |
| 17 | * | fertile | 61.9 | *** | |
| 18 | * | fertile | 103.4 | *** | |
| 19 | * | fertile | 85.4 | *** | |
| 20 | * | fertile | 45.4 | *** | LHS + 1 |
| 21 | * | fertile | 14.9 | *** | |
| 22 | * | fertile | 46.6 | *** | |
| 23 | * | postfertile | 49.3 | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| LH surge was on day 19, therefore repeated testing to commence on day 15 in next cycle. | | | | | |

| CYCLE B 2 | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | * | infertile | 28.9 | | |
| 7 | | infertile | | | |
| 8 | | infertile | | | |
| 9 | | infertile | | | |
| 10 | | infertile | | | |
| 11 | | infertile | | | |
| 12 | | infertile | | *** | |
| 13 | | infertile | | *** | |
| 14 | | infertile | | *** | |
| 15 | * | fertile | 62.0 | *** | |
| 16 | * | fertile | 94.6 | *** | |
| 17 | * | fertile | 58.4 | *** | LHS + 1 |
| 18 | * | fertile | 42.4 | *** | |
| 19 | * | fertile | 60.4 | *** | |
| 20 | * | fertile | 56.0 | *** | |
| 21 | * | postfertile | 35.0 | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Mean LHS from cycles B1 and B2 is day "17.5", therefore repeated testing to commence on day 13 in next cycle. | | | | | |

| CYCLE B 3 | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | * | infertile | 17.2 | | |
| 7 | | infertile | | | |
| 8 | | infertile | | | |
| 9 | | infertile | | | |
| 10 | | infertile | | *** | |
| 11 | | infertile | | *** | |
| 12 | | infertile | | *** | |
| 13 | * | infertile | 23.9 | *** | |
| 14 | * | fertile | 63.8 | *** | |
| 15 | * | fertile | 22.1 | *** | |
| 16 | * | fertile | 65.9 | *** | |
| 17 | * | fertile | 41.2 | *** | LHS + 1 |
| 18 | * | fertile | 7.6 | *** | |
| 19 | * | fertile | 35.3 | *** | |
| 20 | | postfertile | | | |
| 21 | | postfertile | | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Mean LHS from cycles B1 to B3: day 17. Repeated testing commencement day for first routine cycle: day 12. | | | | | |

17

| CYCLE B 4<br>First routine cycle | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | * | infertile | 12.9 | | |
| 7 | | infertile | | | |
| 8 | | infertile | | | |
| 9 | | infertile | | | |
| 10 | | infertile | | | |
| 11 | | infertile | | | |
| 12 | * | infertile | 38.3 | *** | |
| 13 | * | fertile | 70.6 | *** | |
| 14 | * | fertile | 74.6 | *** | |
| 15 | * | fertile | 70.6 | *** | |
| 16 | * | fertile | 49.7 | *** | LHS + 1 |
| 17 | * | fertile | 23.5 | *** | |
| 18 | * | fertile | 29.8 | *** | |
| 19 | | postfertile | | | |
| 20 | | postfertile | | | |
| 21 | | postfertile | | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Days warning of actual ovulation: 4<br>Mean LHS from cycles B1 to B4: day "16.5".<br>Repeated testing commencement day for next cycle: day 11. | | | | | |

| CYCLE B 5<br>Second routine cycle | | | | | |
|---|---|---|---|---|---|
| Day | Test | Phase | E3G value | "Red" Status | Actual Ovulation |
| 1 | | infertile | | | |
| 2 | | infertile | | | |
| 3 | | infertile | | | |
| 4 | | infertile | | | |
| 5 | | infertile | | | |
| 6 | * | infertile | 7.2 | | |
| 7 | | infertile | | | |
| 8 | | infertile | | | |
| 9 | | infertile | | | |
| 10 | | infertile | | | |
| 11 | * | infertile | 14.1 | | |
| 12 | * | infertile | 17.4 | | |
| 13 | * | infertile | 41.3 | *** | |
| 14 | * | infertile | 57.5 | *** | |
| 15 | * | fertile | 42.0 | *** | |
| 16 | * | fertile | 55.4 | *** | |
| 17 | * | fertile | 60.1 | *** | |
| 18 | | fertile | | *** | LHS + 1 |
| 19 | | fertile | | *** | |
| 20 | | fertile | | *** | |
| 21 | | postfertile | | | |
| 22 | | postfertile | | | |
| 23 | | postfertile | | | |
| 24 | | postfertile | | | |
| 25 | | postfertile | | | |
| 26 | | postfertile | | | |
| 27 | | postfertile | | | |
| 28 | | postfertile | | | |
| 29 | | postfertile | | | |
| 30 | | postfertile | | | |
| Days warning of actual ovulation: 5<br>LHS detected on last day at testing. Mean LHS from cycles B1 to B5: day "16.6".<br>Repeated testing commencement day for next cycle: day 11. | | | | | |

**Claims**

1. A method of monitoring the fertility status of an individual female mammalian subject, wherein the concentration of an analyte in body fluid obtainable from the subject is tested at least once during the interval spanning days 1 to 7 inclusive of the current cycle and also later in the current cycle to determine whether a concentration change indicative of imminent ovulation is occurring or has occurred, <u>characterised in that</u>:

    a) the mean numerical day on which actual ovulation has occurred during one or more previous ovulation cycles in the subject is determined;

    b) the at least one test conducted during the interval spanning days 1 to 7 is used to establish a reference concentration value or test signal for said analyte for the current cycle; and

    c) the later testing in the current cycle comprises a series of tests for said analyte conducted during a period of days commencing at least 5 numerical days in advance of said mean numerical ovulation day, and the concentration value or test signal from each of said later tests is compared to the reference concentration value or test signal.

2. A method according to claim 1, <u>characterised in that</u> said later testing is conducted after a cessation of testing following the establishment of said reference concentration value or signal.

3. A method according to claim 1 or claim 2, <u>characterised in that</u> a maximum of 16 body fluid tests are conducted in the current cycle.

4. A method according to claim 1 or claim 2, <u>characterised in that</u> testing is conducted on a maximum of 16 days in the current cycle.

5. A method according to any of the preceding claims, <u>characterised in that</u> said subject is human and said analyte is estradiol or a metabolyte thereof.

6. A method according to claim 5, characterised in that said analyte is estrone-3-glucuronide.

7. A method according to any one of the preceding claims, <u>characterised in that</u> said later testing is conducted during a period of days commencing at least 6 numerical days in advance of the mean numerical ovulation day.

8. A method according to any one of the preceding claims, <u>characterised in that</u> said reference concentration value or test signal is established from a single test.

9. A method according to any one of claims 1 to 7, <u>characterised in that</u> said reference concentration value or test signal is established from test/s conducted during the interval spanning days 4 to 7 inclusive.

10. A method according to claim 9, <u>characterised in that</u> said concentration reference value is established from test/s conducted on day 5 and/or day 6.

11. A method according to claim 8, <u>characterised in that</u> the single test is conducted on day 6.

12. A method according to any one of the preceding claims, <u>characterised in that</u> the body fluid is urine.

13. A method according to claim 5 or claim 6, <u>characterised in that</u> a significant difference between the analyte reference concentration value [r] and a test value [i], indicative of imminent ovulation, is taken to be:

$$1.5 \le \frac{[i]}{[r]} \le 2.5$$

in the case of direct proportionality between the test signal and analyte concentration, or the inverse in the case of inverse proportionality between the test signal and analyte concentration.

14. A method according to claim 13, <u>characterised in that</u> the analyte is estrone-3-glucuronide, and the significant difference in estrone-3-glucuronide concentration [i] indicative of imminent ovulation is taken to be:

$$\frac{[i]}{[r]} \geq 2$$

in the case of direct proportionality between the test signal and estrone-3-glucuronide concentration, or the inverse in the case of inverse proportionality between the test signal and estrone-3-glucuronide concentration.

15. A method according to any one of the preceding claims, <u>characterised in that</u> the body fluid concentration of luteinizing hormone is determined to identify the actual ovulation day in the current cycle.

16. A method according to any one of the preceding claims, <u>characterised in that</u> the mean ovulation day is derived from data collected during at least 3 consecutive previous cycles.

17. A method according to any one of the preceding claims, <u>characterised in that</u> the mean ovulation day is derived from data collected during at least 5 consecutive previous cycles.

18. A method according to any one of the preceding claims, <u>characterised in that</u> the mean ovulation day is derived from data obtained during at least the immediately preceding cycle.

19. A method according to claim 18, <u>characterised in that</u> the mean ovulation day is derived from data obtained from a rolling reference base consisting of a fixed number of consecutive cycles immediately preceding the current cycle.

20. A method according to claim 19, <u>characterised in that</u> the rolling reference base consists of the immediately preceding 3 to 12 cycles.

21. A method according to claim 19, <u>characterised in that</u> the rolling reference base consists of the immediately preceding 5 or 6 cycles.

22. A method according to any one of the preceding claims, <u>characterised in that</u> the end of the fertile phase is declared from knowledge of the luteinizing hormone surge in the current cycle.

23. A method according to claim 22, <u>characterised in that</u> the end of the fertile phase is taken to occur on the fourth day following luteinizing hormone surge detection.

24. A method according to any one of claims 1 to 21, <u>characterised in that</u> the end of the fertile phase is declared from knowledge of the concentration of estradiol or a metabolite thereof in the current cycle.

25. A method according to claim 24, <u>characterised in that</u> the end of the fertile phase is declared from knowledge of the peak concentration of estradiol or a metabolite thereof in the current cycle, this peak being defined as the first occasion in the cycle when

$$\frac{[i]}{[r]} > 2.5, \text{ preferably} \geq 3,$$

is detected in the case of direct proportionality between the test signal and estradiol/metabolite concentration, or the inverse in the case of inverse proportionality between the test signal and estradiol/metabolite concentration.

26. A method according to claim 25, <u>characterised in that</u> the end of the fertile phase is taken to occur on the eighth day following the estradiol/metabolite peak detection.

27. A method according to any one of claims 1 to 21, <u>characterised in that</u> the body fluid concentration of pregnanediol-3-glucuronide is measured.

28. A method according to claim 27, <u>characterised in that</u> the end of the fertile phase is declared after the detection of an elevated pregnanediol-3-glucuronide concentration.

29. A method according to any one of the preceding claims, <u>characterised in that</u> it solely utilises body fluid analyte concentration data.

30. A method according to any one of the preceding claims, <u>characterised in that</u> said series of test for said analyte are conducted on an at least daily basis.

31. A method according to claim 1, <u>characterised in that</u>:

a) the user is provided with a plurality of disposable body fluid testing devices for testing for said analyte, said plurality being at least 7 but not greater than 16; and

b) the user uses all of said provided testing devices during a single ovulation cycle, irrespective of whether an indication of imminent ovulation has been obtained before all of said provided testing devices have been used.

32. A method according to claim 31, <u>characterised in that</u> the user performs one test on day 6, and uses all of the remaining testing devices on a daily basis during a repeated testing period.

**Patentansprüche**

1. Verfahren zur Überwachung des Fruchtbarkeitsstatus eines jeweiligen weiblichen Individuums aus der Gruppe der Säuger, wobei die Konzentration eines Analyten in einer von dem Individuum erhältlichen Körperflüssigkeit mindestens einmal während der die Tage 1 bis 7 einschließenden Zeitspanne des laufenden Zyklus und auch später im laufenden Zyklus getestet wird, um zu bestimmen, ob eine die unmittelbar bevorstehende Ovulation anzeigende Konzentrationsänderung auftritt oder aufgetreten ist, dadurch gekennzeichnet, daß:

a) der durchschnittliche numerische Tag bestimmt wird, an dem die eigentliche Ovulation während eines oder mehrerer vorhergehender Ovulationszyklen in dem Individuum aufgetreten ist;

b) der zumindest eine Test, der während der die Tage 1 bis 7 einschließenden Zeitspanne durchgeführt wurde, verwendet wird, um einen Referenz-Konzentrationswert oder -Testsignal für den Analyten für den laufenden Zyklus zu etablieren; und

c) das spätere Testen im laufenden Zyklus eine Serie von Tests auf den Analyten umfaßt, die während eines Zeitraums von Tagen, beginnend mindestens 5 numerische Tage vor dem durchschnittlichen numerischen Tag der Ovulation, durchgeführt werden und der Konzentrationswert oder das Testsignal von jedem dieser späteren Tests mit dem Referenz-Konzentrationswert oder -Testsignal verglichen wird.

2. Verfahren nach Anspruch 1, dadurch gekennzeichnet, daß das letztgenannte Testen durchgeführt wird, nachdem nach Etablieren des Referenz-Konzentrationswertes oder -Signals das Testen eingestellt worden ist.

3. Verfahren nach Anspruch 1 oder Anspruch 2, dadurch gekennzeichnet, daß maximal 16 Körperflüssigkeitstests in dem laufenden Zyklus durchgeführt werden.

4. Verfahren nach Anspruch 1 oder Anspruch 2, dadurch gekennzeichnet, daß das Testen an maximal 16 Tagen im laufenden Zyklus durchgeführt wird.

5. Verfahren nach mindestens einem der vorhergehenden Ansprüche, dadurch gekennzeichnet, daß das Individuum ein Mensch ist und der Analyt Östradiol oder ein Metabolit von diesem.

6. Verfahren nach Anspruch 5, dadurch gekennzeichnet, daß der Analyt Östron-3-glucuronid ist.

7. Verfahren nach mindestens einem der vorhergehenden Ansprüche, dadurch gekennzeichnet, daß das spätere Testen während eines Zeitraums von Tagen durchgeführt wird, der mindestens 6 numerische Tage vor dem durchschnittlichen numerischen Tag der Ovulation beginnt.

8. Verfahren nach mindestens einem der vorhergehenden Ansprüche, dadurch gekennzeichnet, daß der Referenz-Konzentrationswert oder das -Testsignal aus einem einzigen Test etabliert wird.

9. Verfahren nach mindestens einem der Ansprüche 1 bis 7, dadurch gekennzeichnet, daß der Referenz-Konzentrationswert oder das -Testsignal aus einem oder mehreren Tests etabliert wird, der/die während der die Tage 4 bis 7 einschließenden Zeitspanne durchgeführt wird/werden.

10. Verfahren nach Anspruch 9, dadurch gekennzeichnet, daß der Referenz-Konzentrationswert aus einem oder mehreren Tests etabliert wird, der/die an Tag 5 und/oder Tag 6 durchgeführt wird/werden.

11. Verfahren nach Anspruch 8, dadurch gekennzeichnet, daß der einzelne Test an Tag 6 durchgeführt wird.

12. Verfahren nach mindestens einem der vorhergehenden Ansprüche, dadurch gekennzeichnet, daß die Körperflüssigkeit Urin ist.

13. Verfahren nach Anspruch 5 oder Anspruch 6, dadurch gekennzeichnet, daß als signifikante, die unmittelbar bevorstehende Ovulation anzeigende Differenz zwischen dem Referenz-Analytkonzentrationswert [r] und einem Testwert [i] angenommen wird:

$$1{,}5 \leq \frac{[i]}{[r]} \leq 2{,}5,$$

für den Fall einer direkten Proportionalität zwischen dem Testsignal und der Analytkonzentration oder der Kehrwert für den Fall einer inversen Proportionalität zwischen dem Testsignal und der Analytkonzentration.

14. Verfahren nach Anspruch 13, dadurch gekennzeichnet, daß der Analyt Östron-3-glucuronid ist, und die signifikante, das unmittelbar bevorstehende Ovulationsereignis anzeigende Differenz in der Östron-3-glucuronid Konzentration [i] angenommen wird als:

$$\frac{[i]}{[r]} \geq 2$$

für den Fall einer direkten Proportionalität zwischen dem Testsignal und der Östron-3-glucuronid-Konzentration oder der Kehrwert für den Fall einer inversen Proportionalität zwischen dem Testsignal und der Östron-3-glucuronid-Konzentration.

15. Verfahren nach mindestens einem der vorhergehenden Ansprüche, dadurch gekennzeichnet, daß die Konzentration des luteinisierenden Hormons in Körperflüssigkeit zur Identifizierung des eigentlichen Ovulationstages im laufenden Zyklus bestimmt wird.

16. Verfahren nach mindestens einem der vorhergehenden Ansprüche, dadurch gekennzeichnet, daß der durchschnittliche Ovulationstag aus Daten abgeleitet wird, die während mindestens 3 aufeinanderfolgender vorhergehender Zyklen gesammelt wurden.

17. Verfahren nach mindestens einem der vorhergehenden Ansprüche, dadurch gekennzeichnet, daß der durchschnittliche Ovulationstag aus Daten abgeleitet wird, die während mindestens 5 aufeinanderfolgender vorhergehender Zyklen gesammelt wurden.

18. Verfahren nach mindestens einem der vorhergehenden Ansprüche, dadurch gekennzeichnet, daß der durchschnittliche Ovulationstag aus Daten abgeleitet wird, die während zumindest des unmittelbar vorhergehenden Zyklus erhalten wurden.

19. Verfahren nach Anspruch 18, dadurch gekennzeichnet, daß der durchschnittliche Ovulationstag aus Daten abgeleitet wird, die aus einer beweglichen Referenzbasis erhalten wurden, die aus einer festen Zahl fortlaufender, dem laufenden Zyklus unmittelbar vorhergehender Zyklen besteht.

20. Verfahren nach Anspruch 19, dadurch gekennzeichnet, daß die bewegliche Referenzbasis aus den unmittelbar vorhergehenden 3 bis 12 Zyklen besteht.

21. Verfahren nach Anspruch 19, dadurch gekennzeichnet, daß die bewegliche Referenzbasis aus den unmittelbar vorhergehenden 5 bis 6 Zyklen besteht.

22. Verfahren nach mindestens einem der vorhergehenden Ansprüche, dadurch gekennzeichnet, daß das Ende der fruchtbaren Phase aufgrund der Kenntnis des Anstiegs des luteinisierenden Hormons im laufenden Zyklus angegeben wird.

23. Verfahren nach Anspruch 22, dadurch gekennzeichnet, daß man das Auftreten des Endes der fruchtbaren Phase am vierten Tag nach Detektion des Anstiegs des luteinisierenden Hormons annimmt.

24. Verfahren nach mindestens einem der Ansprüche 1 bis 21, dadurch gekennzeichnet, daß das Ende der fruchtbaren Phase aufgrund der Kenntnis der Konzentration von Östradiol oder einem seiner Metaboliten im laufenden Zyklus angegeben wird.

25. Verfahren nach Anspruch 24, dadurch gekennzeichnet, daß das Ende der fruchtbaren Phase aufgrund der Kenntnis der Peak-Konzentration von Östradiol oder einem seiner Metaboliten im laufenden Zyklus angegeben wird, wobei dieser Peak als das erste Ereignis im Zyklus definiert wird, wenn

$$\frac{[i]}{[r]} > 2,5, \text{ vorzugsweise } \geq 3,$$

detektiert wird für den Fall einer direkten Proportionalität zwischen dem Testsignal und der Östradiol/Metabolit-Konzentration oder der Kehrwert für den Fall einer inversen Proportionalität zwischen dem Testsignal und der Östradiol/Metabolit-Konzentration.

26. Verfahren nach Anspruch 25, dadurch gekennzeichnet, daß man das Ende der fruchtbaren Phase für den achten Tag nach der Detektion des Östradiol/Metaboliten-Peaks annimmt.

27. Verfahren nach mindestens einem der Ansprüche 1 bis 21, dadurch gekennzeichnet, daß die Konzentration von Pregnandiol-3-glucuronid in Körperflüssigkeit gemessen wird.

28. Verfahren nach Anspruch 27, dadurch gekennzeichnet, daß das Ende der fruchtbaren Phase nach Detektion einer erhöhten Pregnandiol-3-glucuronid-Konzentration angegeben wird.

29. Verfahren nach mindestens einem der vorhergehenden Ansprüche, dadurch gekennzeichnet, daß es alleine Körperflüssigkeitsanalyten-Konzentrationswerte verwendet.

30. Verfahren nach mindestens einem der vorhergehenden Ansprüche, dadurch gekennzeichnet, daß die Serie von Tests auf den Analyten zumindest in täglicher Abfolge durchgeführt wird.

31. Verfahren nach Anspruch 1, dadurch gekennzeichnet, daß

a) der Verwender mit einer Mehrzahl von Einweg-Körperflüssigkeit-Testvorrichtungen zum Testen auf den Analyten ausgestattet wird, wobei die Anzahl mindestens 7 beträgt, aber nicht größer als 16 ist; und

b) der Verwender alle bereitgestellten Testvorrichtungen während eines einzelnen Ovulationszyklus verwendet, unabhängig davon, ob eine Anzeige für eine unmittelbar bevorstehende Ovulation erhalten worden ist, bevor alle bereitgestellten Testvorrichtungen verwendet worden sind.

32. Verfahren nach Anspruch 31, dadurch gekennzeichnet, daß der Verwender einen Test an Tag 6 ausführt und alle verbleibenden Testvorrichtungen in täglicher Abfolge während einer wiederholten Testperiode verwendet.

**Revendications**

1. Procédé de suivi de l'état de fertilité chez un sujet mammifère de sexe féminin, dans lequel on teste la concentration d'un analyte dans un liquide corporel que l'on peut obtenir chez le sujet au moins une fois au cours de l'intervalle couvrant les jours 1 à 7 compris du cycle en cours ainsi que plus tard dans le cycle en cours pour déterminer si oui ou non une modification de concentration indiquant une ovulation imminente se produit ou s'est produite, caractérisé en ce que :

a) on détermine le jour numérique moyen auquel une ovulation effective s'est produite au cours d'un ou plusieurs cycles d'ovulation antérieure chez le sujet ;

b) on utilise l'épreuve conduite au moins un jour dans l'intervalle couvrant les jours 1 à 7 afin d'établir une valeur de concentration de référence ou un signal de test pour ledit analyte portant sur le cycle en cours ; et

c) la dernière épreuve dans le cycle en cours comprend une série de tests pour ledit analyte conduits au cours d'une période de jours commençant au moins 5 jours numériques en avance dudit jour d'ovulation numérique moyen, et l'on compare la valeur de concentration ou le signal test provenant de chacun desdits tests ultérieurs à la valeur de concentration de référence ou au signal de test.

2. Procédé selon la revendication 1, caractérisé en ce que ledit essai ultérieur effectué après une cessation des épreuves suivant l'établissement de ladite valeur ou dudit signal de concentration de référence.

3. Procédé selon la revendication 1 ou la revendication 2, caractérisé en ce qu'on effectue un maximum de 16 tests de liquide corporel dans le cycle en cours.

4. Procédé selon la revendication 1 ou la revendication 2, caractérisé en ce qu'on conduit l'épreuve pendant un maximum de 16 jours dans le cycle en cours.

5. Procédé selon l'une quelconque des revendications précédentes, caractérisé en ce que ledit sujet est humain et en ce que ledit analyte est l'oestradiol ou un de ses métabolites.

6. Procédé selon la revendication 5, caractérisé en ce que ledit analyte est l'oestrone-3-glucuronide, E3G.

7. Procédé selon une quelconque des revendications précédentes, caractérisé en ce que lesdites épreuves ultérieures sont conduites pendant une période de jours commençant au moins 6 jours numériques avant le jour d'ovulation numérique moyen.

8. Procédé selon l'une quelconque des revendications précédentes, caractérisé en ce qu'on établit ladite valeur de concentration de référence ou ledit signal de test à partir d'une seule épreuve.

9. Procédé selon l'une quelconque des revendications 1 à 7, caractérisé en ce que ladite valeur de concentration de référence ou ledit signal de test est établi à partir d'une ou plusieurs épreuves conduites au cours de l'intervalle couvrant les jours couvrant les jours 4 à 7 compris.

10. Procédé selon la revendication 9, caractérisé en ce que ladite valeur de concentration de référence est établie à partir d'un ou plusieurs tests conduits au jour5 et/ou 6.

11. Procédé selon la revendication 8, caractérisé en ce que le test unique est effectué au jour 6.

12. Procédé selon l'une quelconque des revendications précédentes, caractérisé en ce que le liquide corporel est l'urine.

13. Procédé selon la revendication 5 ou la revendication 6, caractérisé en ce qu'on considère comme une différence significative entre la valeur de concentration de référence de l'analyte [r] et une valeur de test [i], indiquant une ovulation imminente, le nombre

$$1,5 \leq \frac{[i]}{[r]} \leq 2,5$$

dans le cas d'une proportionnalité directe entre le signal de test et la concentration d'analyte, ou l'inverse dans le cas d'une proportionnalité inverse entre le signal de test et la concentration d'analyte.

14. Procédé selon la revendication 13, caractérisé en ce que l'analyte est l'oestrone-3-glucoronide, et en ce que la différence significative de concentration d'oestrone-3-glucoronide [i] indiquant une ovulation imminente est prise comme étant :

$$\frac{[i]}{[r]} \geq 2$$

dans la cas d'une proportionnalité directe entre le signal de test et la concentration d'E3G, ou l'inverse dans le cas d'une proportionnalité inverse entre le signal de test et la concentration d'E3G.

15. Procédé selon l'une quelconque des revendications précédentes, caractérisé en ce qu'on détermine que la concentration dans le liquide corporel de l'hormone lutéinisante, estrone-3-glucoronide, identifie le jour d'ovulation effectif dans le cycle en cours.

16. Procédé selon l'une quelconque des revendications précédentes, caractérisé en ce que le jour moyen d'ovulation est obtenu à partir de données recueillies au cours d'au moins trois cycles antérieurs consécutifs.

17. Procédé selon l'une quelconque des revendications précédentes, caractérisé en ce que le jour moyen d'ovulation est obtenu à partir de données recueillies pendant au moins 5 cycles antérieurs consécutifs.

18. Procédé selon l'une quelconque des revendications précédentes, caractérisé en ce que le jour moyen d'ovulation est déterminé à partir de données obtenues au cours d'au moins le cycle immédiatement précédent.

19. Procédé selon la revendication 18, caractérisé en ce que le jour moyen d'ovulation est déduit de données obtenues à partir d'une base de référence mobile constituée d'un nombre fixe de cycles consécutifs immédiatement précédant le cycle en cours.

20. Procédé selon la revendication 19, caractérisé en ce que la base de référence mobile se compose des 3 à 12 cycles immédiatement précédents.

21. Procédé selon la revendication 19, caractérisé en ce que la base de référence mobile se compose des 5 ou 6 cycles immédiatement précédents.

22. Procédé selon l'une quelconque des revendications précédentes, caractérisé en ce que la fin de la phase fertile est déclarée à partir de la connaissance de la montée de l'hormone luteinisante dans le cycle en cours.

23. Procédé selon la revendication 22, caractérisé en ce que la fin de la phase fertile est prise comme survenant le quatrième jour suivant la détection de la montée de l'hormone luteinisante.

24. Procédé selon l'une quelconque des revendications 1 à 21, caractérisé en ce que la fin de la phase fertile est déclarée à partir de la connaissance de la concentration d'oestradiol ou d'un de ses métabolites dans le cycle en cours.

25. Procédé selon la revendication 24, caractérisé en ce que la fin de la phase fertile est déclarée à partir de la connaissance du pic de concentration d'oestradiol ou d'un de ses métabolites dans le cycle en cours, ce pic étant défini comme étant la première occasion dans le cycle où

$$\frac{[i]}{[r]} > 2,5, \text{ de préférence} \geq 3$$

est détecté dans le cas d'une proportionnalité directe entre le signal de test et la concentration d'oestradiol/métabolite, ou l'inverse dans le cas d'une proportionnalité inverse entre le signal de test et la concentration d'oestradiol/métabolite.

26. Procédé selon la revendication 24, caractérisé en ce qu'on prend la fin de la phase fertile comme apparaissant le huitième jour suivant la détection du pic d'oestradiol/métabolite.

27. Procédé selon l'une quelconque des revendications 1 à 21, caractérisé en ce qu'on mesure la concentration, dans un liquide corporel, du prégnanediol-3-glucuronide.

28. Procédé selon la revendication 27, caractérisé en ce qu'on déclare la fin de la phase fertile après la détection d'une concentration élevée de prégnanediol-3-glucuronide.

29. Procédé selon l'une quelconque des revendications précédentes, caractérisé en ce qu'il utilise uniquement un analyte de liquide corporel.

30. Procédé selon l'une quelconque des revendications précédentes, caractérisé en ce qu'on conduit ladite série de tests pour ledit analyte sur une base au moins quotidienne.

31. Procédé selon la revendication 1, caractérisé en ce que :

   a) l'utilisatrice est munie de plusieurs dispositifs de test de liquide corporel jetable pour tester ledit analyte, ladite pluralité étant un nombre au moins étal à 7 mais ne dépassant pas 12 ; et
   b) l'utilisatrice emploie la totalité desdits dispositifs de test fournis au cours d'un seul cycle d'ovulation, que l'on ait obtenu ou non une indication d'une ovulation imminente avant que la totalité desdits dispositifs de test fournis aient été utilisés.

32. Procédé selon la revendication 31, caractérisé en ce que l'utilisatrice effectue un test au jour 6, et utilise la totalité du dispositif de test restant sur une base quotidienne, au cours d'une période d'épreuves répétées.